(19) Europäisches Patentamt
European Patent Office
Office européen des brevets

(11) Veröffentlichungsnummer: **0 173 870**
**A1**

(12) # EUROPÄISCHE PATENTANMELDUNG

(21) Anmeldenummer: **85109806.1**

(22) Anmeldetag: **05.08.85**

(51) Int. Cl.⁴: **C 07 D 241/12**
C 08 G 18/78

(30) Priorität: **16.08.84 DE 3429962**

(43) Veröffentlichungstag der Anmeldung:
**12.03.86 Patentblatt 86/11**

(84) Benannte Vertragsstaaten:
**BE DE FR GB IT**

(71) Anmelder: **BAYER AG**
**Konzernverwaltung RP Patentabteilung**
**D-5090 Leverkusen 1 Bayerwerk(DE)**

(72) Erfinder: **Scholl, Hans-Joachim, Dr.**
**Rudolf-Sohm-Strasse 28**
**D-5000 Köln 60(DE)**

(54) **2,5-Di-(4'isocyanatobutyl)-3,6-dimethylpyrazin, ein Verfahren zu seiner Herstellung und seine Verwendung bei der Herstellung von Polyurethankunststoffen.**

(57) 2,5-Di-(4'-isocyanatobutyl)-3,6-dimethylpyrazin, ein neues Diisocyanat mit eingebauten tert. Stickstoffatomen, ein Verfahren zu seiner Herstellung durch Phosgenierung des ihm zugrundeliegenden Diamins und seine Verwendung als Aufbaukomponente bei der Herstellung von Polyurethankunststoffen nach dem Isocyanat-Polyadditionsverfahren.

EP 0 173 870 A1

0173870

BAYER AKTIENGESELLSCHAFT 5090 Leverkusen, Bayerwerk
Konzernverwaltung RP
Patentabteilung Wr/Em-c

2,5-Di-(4'-isocyanatobutyl)-3,6-dimethylpyrazin, ein Verfahren zu seiner Herstellung und seine Verwendung bei der Herstellung von Polyurethankunststoffen

Gegenstand der Erfindung ist 2,5-Di-(4'-isocyanatobutyl)-3,6-dimethylpyrazin (DIDP) der Formel

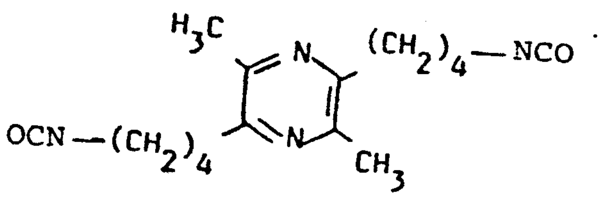

Gegenstand der Erfindung ist auch ein Verfahren zur Herstellung von 2,5-Di-(4'-isocyanatobutyl)-3,6-dimethylpyrazin, dadurch gekennzeichnet, daß man 2,5-Di-(4'-aminobutyl)-3,6-dimethylpyrazin (DADP) bzw. dessen Additionsverbindungen mit Kohlendioxid oder Chlorwasserstoff phosgeniert.

Gegenstand der Erfindung ist schließlich auch die Verwendung von 2,5-Di-(4'-isocyanatobutyl)-3,6-dimethylpyrazin als Isocyanatkomponente bei der Herstellung von Polyurethankunststoffen nach dem Isocyanat-Polyadditionsverfahren.

DIDP ist ein neues Diisocyanat mit aliphatisch gebundenen Isocyanatgruppen und eingebauten tert.-Stickstoffatomen. DIDP stellt daher ein wertvolles Ausgangsmaterial zur Herstellung von Polyurethankunststoffen nach dem Isocyanat-Polyadditionsverfahren dar. Durch die Verwendung DIDP als Isocyanatkomponente bei der Herstellung von Polyurethankunststoffen werden in das Polyurethangerüst Pyrazin-Struktureinheiten eingebaut, die als Hartsegmente wirken und damit dem Polyurethan wertvolle mechanische Eigenschaften verleihen, und die darüber hinaus durch Quaternierung bzw. durch Neutralisation in ternäre bzw. quaternäre Ammoniumgruppen überführbar sind, so daß die eingebautes DIDP aufweisenden Polyurethane auf einfache Weise in Wasser dispergierbare Polyadditionsprodukte überführt werden können.

Als Ausgangsmaterial für das erfindungsgemäße Verfahren dient DADP der Formel

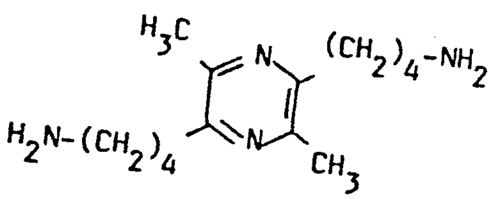,

welches in an sich bekannter Weise durch Phosgenierung in DIDP überführbar ist. Hierbei kann sowohl nach dem bekannten "Basenverfahren", als auch nach dem bekannten "Chlorwasserstoff-Verfahren" als auch nach dem bekannten "Kohlendioxid-Verfahren" gearbeitet werden. Als Reaktionsmedium dient in allen Fällen vorzugsweise Chlorbenzol

und/oder auch o-Dichlorbenzol. Die Phosgeniertemperatur liegt im allgemeinen bei -10°C - +180°C, vorzugsweise bei 0°C - 150°C. Bei dem genannten "Basenverfahren" wird das freie Diamin direkt mit Phosgen zur Reaktion gebracht. Hierbei empfiehlt sich die Anwendung des Prinzips der "Kalt-Heiß-Phosgenierung", d.h. es wird zunächst bei einer vergleichsweise niederen Temperatur innerhalb der genannten Temperaturbereiche durch Umsetzung der Base mit dem Phosgen das entsprechende Carbamidsäurechlorid hergestellt, welches anschließend unter fortgesetzter Phosgenzugabe bei erhöhter Temperatur innerhalb der genannten Temperaturbereiche in DIDP überführt wird. Bei Anwendung des "Chlorwasserstoff-Verfahrens" bzw. des "Kohlendioxid-Verfahrens" wird vorzugsweise zunächst bei einer niederen Temperatur innerhalb der genannten Temperaturbereiche das entsprechende Addukt aus DADP und HCl bzw. $CO_2$ hergestellt, welches anschliessend unter fortgesetzter Phosgenzugabe bei einer erhöhten Temperatur innerhalb der genannten Temperaturbereiche in DIDP überführt wird. Bei allen Ausführungsformen des erfindungsgemäßen Verfahrens erfolgt die Reindarstellung von DIDP vorzugsweise durch destillative Aufarbeitung des Reaktionsansatzes, der gegebenenfalls vor der destillativen Aufarbeitung durch Filtration von festen Nebenprodukten befreit worden ist. DIDP fällt hierbei als, bei 180-185°C/1mbar destillierbare, bei Raumtemperatur eine geringe Neigung zur Kristallisation aufweisende, Flüssigkeit an.

Die Herstellung des als Ausgangsmaterial beim erfindungsgemäßen Verfahren einzusetzenden DADP kann beispielsweise durch saure Hydrolyse von 3,7-Bisacetamino-heptanon-2 und anschließende Cyclokondensation unter gleichzeitiger Wasserstoffabspaltung des als Hydrolyseprodukt (in der Salzform) anfallenden 3,7-Diamino-heptanon-2 unter dem Einfluß starker Basen hergestellt werden.

Zur sauren Hydrolyse des genannten Ausgangsmaterials können beispielsweise Lösungen von 3,7-Bisacetamino-heptanon-2 in Wasser bei Rückflußtemperatur, d.h. bei ca. 100°C mit starken Säuren behandelt werden. Als starke Säuren kommen beispielsweise organische oder anorganische Säuren wie z.B. Toluolsulfonsäure, Schwefelsäure oder vorzugsweise Salzsäure in Betracht. Die Säure wird im allgemeinen in einer Menge von 2 - 12 Mol, vorzugsweise 3 - 6 Mol pro Mol 3,7-Bisacetamino-heptanon-2 eingesetzt. Die saure Hydrolyse ist im allgemeinen nach einer Reaktionsdauer von 2 - 6 Stunden beendet. Es ist bevorzugt, bei der sauren Hydrolyse eine flüchtige Säure wie Salzsäure einzusetzen, da in einem solchen Falle die überschüssige Säure bzw. der Hauptteil der überschüssigen Säure vor der anschließenden, in basischem Milieu stattfindenden Kondensationsreaktion destillativ aus dem Reaktionsgemisch entfernt werden kann und daher nicht neutralisiert werden muß.

Das saure Hydrolysegemisch wird im Anschluß an seine Herstellung, vorzugsweise nach destillativer Abtrennung von leichtflüchtigen Bestandteilen inklusive der Hauptmenge des Wassers und der vorzugsweise flüchtigen Säure

bis zur stark basischen Reaktion mit starken Basen versetzt. Geeignete starke Basen sind organische oder anorganische Basen wie z.B. Triethylamin, Natronlauge, Kalilauge oder die entsprechenden Alkalicarbonate. Bevorzugt werden starke anorganische Basen wie Natronlauge oder Kalilauge verwendet. Im allgemeinen wird ein 0,5- bis 10-, vorzugsweise 1- bis 6-molarer Überschuß der Base pro Mol an eingesetztem 3,7-Bisacetamino-heptanon-2 verwendet, wobei unter "Überschuß" die Menge an Base zu verstehen ist, die über die Menge an Base hinausgeht, die zur Neutralisation der im Reaktionsgemisch vorliegenden freien und an das Diamin gebundenen Säure erforderlich ist. Nach erfolgter Basenzugabe wird das basische Reaktionsgemisch im allgemeinen innerhalb des Temperaturbereiches von 0 - 60°C, vorzugsweise bei Raumtemperatur gehalten. Die hierbei spontan ablaufende Cyclokondensationsreaktion unter gleichzeitiger Abspaltung von Wasserstoff führt unmittelbar zum erwünschten DADP. Die Aufarbeitung des nach Beendigung der Reaktion vorliegenden Reaktionsgemisches kann beispielsweise so erfolgen, daß man mit wasserunlöslichen Lösungsmitteln, wie beispielsweise Diethylether, Benzol, Toluol oder Chlorbenzol, das Reaktionsprodukt aus der stark basischen, salzhaltigen wäßrigen Lösung extrahiert und nachfolgend vom Extraktionsmittel befreit.

Die geschilderte Herstellung von DADP kann durch folgendes Formelschema erläutert werden:

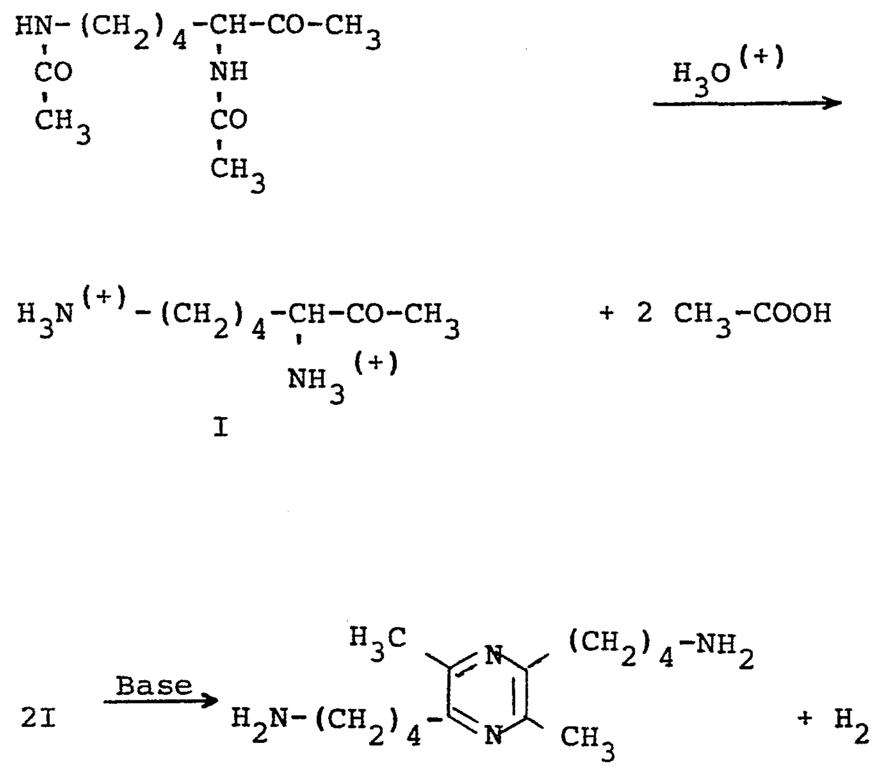


DADP

Das zur Herstellung des DADP angesetzte 3,7-Bisacetamino-heptanon-2 kann beispielsweise gemäß der Verfahrensweise der deutschen Patentanmeldung P 34 25 814.0 erhalten werden. Hierbei wird L-Lysin in freier Form und/oder in Form seines Chlorwasserstoff-Salzes mit Essigsäureanhydrid in Gegenwart einer tertiären organischen Aminbase und unter Zusatz eines 4-Aminopyridin-Derivates umgesetzt.

0173870

Bezogen auf 1 Mol L-Lysin und/oder L-Lysin-hydrochlorid können beispielsweise 4 bis 10 Mol Essigsäureanhydrid eingesetzt werden. Vorzugsweise beträgt diese Menge 6 bis 8 Mol.

Als tertiäre organische Aminbasen kommen beispielsweise Trialkylaminie in Frage, die Alkylgruppen mit 2 bis 6 C-Atomen enthalten. Bevorzugt wird Triethylamin eingesetzt. Bezogen auf 1 Mol L-Lysin und/oder L-Lysin-hydrochlorid können beispielsweise 3 bis 8 Mol einer tertiären organischen Aminbase eingesetzt werden. Vorzugsweise beträgt diese Menge 4 bis 6 Mol.

Als 4-Aminopyridin-Derivate kommen beispielsweise solche der Formel (II) in Frage

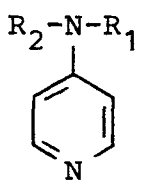

(II),

in der

$R_1$ und $R_2$ unabhängig voneinander für einen einbindigen $C_1$- bis $C_6$-Alkylrest oder $R_1$ und $R_2$ gemeinsam für einen zweibindigen $C_3$- bis $C_5$-Alkylrest stehen.

Bevorzugt wird N,N-Dimethyl-4-aminopyridin oder 4-Pyrrolidino-pyridin eingesetzt. Bezogen auf L-Lysin und/oder L-Lysin-hydrochlorid können beispielsweise 0,1 bis 5

Gew.-% eines 4-Aminopyridin-Derivates eingesetzt werden. Vorzugsweise beträgt diese Menge 0,5 bis 3 Gew.-%.

Die Umsetzung wird im allgemeinen im Temperaturbereich von 20 bis 100°C, vorzugsweise 30 bis 70°C durchgeführt.

Die Aufarbeitung ist des nach Beendigung der Reaktion vorliegenden Reaktionsgemisches kann beispielsweise so erfolgen, daß man, falls das Hydrochlorid der eingesetzten tertiären organischen Aminbase ausgefallen ist, dieses abfiltriert, aus dem Filtrat oder, falls keine festen Bestandteile vorliegen, aus dem gesamten Reaktionsgemisch niedrigsiedende Anteile im Vakuum entfernt, das verbleibende Rohprodukt in geeignetes Lösungsmittel einrührt, wie z.B. in Essigsäureethylester oder Toluol und den entstehenden Niederschlag abfiltriert, wäscht und trocknet.

Die Herstellung von 3,7-Bisacet-amino-heptanon-2 nach diesem Verfahrensprinzip wird im übrigen im nachfolgenden Beispiel 1 erläutert.

Die Herstellbarkeit von DIDP nach dem erfindungsgemäßen Verfahren in guten Ausbeuten und hoher Selektivität ist überraschend, da das als Ausgangsmaterial eingesetzte DADP heterocyclisch gebundene, basische Ringstickstoffatome aufweist, von denen hätte erwartet werden sollen, daß sie insbesondere bei den zur Anwendung gelangenden hohen Phosgenier- und Aufarbeitungstemperaturen unerwünschte Nebenreaktionen der gebildeten Isocyanatgruppen katalysieren.

DIDP stellt eine wertvolle Aufbaukomponente für die Herstellung von Polyurethankunststoffen dar. Das neue Diisocyanat kann anstelle oder zusammmen mit den aus der Polyurethanchemie bekannten Diisocyanaten bei allen bekannten Verfahren zur Herstellung von Polyurethankunststoffen eingesetzt werden. Da das neue Diisocyanat eingebaute basische Stickstoffatome aufweist, eignet es sich besonders gut zur Herstellung von selbstdispergierbaren kationisch modifizierten Polyurethanen unter Mitverwendung der hierzu üblicherweise eingesetzten Ausgangsmaterialien, da die eingebauten basischen Stickstoffatome durch eine einfache Quaternierungsreaktion oder Neutralisationsreaktion in hydrophile Ammoniumgruppen überführbar sind. Die Herstellung derartiger kationisch modifizierter Polyurethane kann beispielsweise in Analogie zur Lehre der US-PS 3 479 310 erfolgen.

In den nachfolgenden Beispielen beziehen sich alle Prozentangaben auf Gewichtsprozente.

Beispiel 1 (Herstellung von 3,7-Bisacetamino-heptanon-2)

In eine Mischung aus 1230 g Essigsäureanhydrid und 810 g Triethylamin wurden 365 g L-Lysinhydrochlorid und 5 g 4-Pyrrolidino-pyridin unter Rühren eingetragen.

Die Reaktionstemperatur wurde bei 40 bis 50°C bis zur Beendigung der Gasentwicklung gehalten. Dann wurde das ausgefallene Triethylaminohydrochlorid abfiltriert und das Filtrat im Vakuum von niedrigsiedenden Anteilen befreit.

Das verbleibende Rohprodukt wurde in 3 Liter Ethylacetat eingerührt, der entstehende Niederschlag abgesaugt, mit Ethylacetat nachgewaschen und getrocknet. Es wurden 402 g (88 % der Theorie) 3,7-Bisacetamino-heptanon-2 mit einem Schmelzpunkt von 111 bis 113°C erhalten.

Analyse:

| | % C | % H | % N |
|---|---|---|---|
| gefunden: | 57,7 | 8,7 | 12,2 |
| Theorie: | 57,9 | 8,8 | 12,3 |

Beispiel 2 (Herstellung von DADP)

114 g 3,7-Bisacetamino-heptanon-2 wurden in 0,4 l 18 %iger Salzsäure 5 h unter Rückflußbedingungen der Hydrolyse unterworfen. Danach wurden im Vakuum flüchtige Bestandteile bis auf ca. 200 ml Rohlösung abgetrennt und in die verbleibende Rohlösung unter

starkem Rühren und Eiskühlung 500 g 45 %ige Natronlauge so eingetropft, daß die Temperatur 20°C nicht überstieg. Man rührte 30 Minuten nach, schüttelte die wäßrige Phase mit Chlorbenzol portionsweise aus, bis das gesamte entstandene Rohamin extrahiert war (ca. 0,5 l Chlorbenzol), trocknete über Kaliumcarbonat und erhielt nach Abtrennung des Chlorbenzols 58 g Rohamin (gaschromatographische Reinheit 94 %), welches als solches oder auch nach destillativer Reinigung für die Phosgenierung geeignet ist.

58 g Rohamin wurden einer Dünnschichtdestillation (Heizbad 210°C, 1 mbar, Übergang bei 170 bis 175°C) unterworfen. Man erhielt 54,3 g 2,5-Di-(4'-aminobutyl)-3,6-dimethylpyrazin (DADP) als gelbliche Flüssigkeit (Gesamtausbeute 87 %).

Analyse:

| | % C | % H | % N |
|---|---|---|---|
| gefunden: | 67,0 | 10,4 | 22,1 |
| Theorie: | 67,2 | 10,4 | 22,4 |

(bezogen auf $C_{14}H_{26}N_4$).

Beispiel 3 (Herstellung von DADP)

114 g 3,7-Bisacetamino-heptanon-2 wurden in 0,5 l 18 %iger Salzsäure 6 h unter Rückflußbedingungen der Hydrolyse unterworfen. Anschließend wurden im Vakuum

flüchtige Bestandteile abgetrennt, man erhielt 110 g Rückstand, der mit 100 ml Aceton gewaschen wurde. Man versetzte den Rückstand mit 100 ml Wasser und tropfte unter starkem Rühren und Eiskühlung 400 g 45 %ige Natronlauge bei 0 - 10°C zu. Danach wurde 1 Stunde bei Raumtemperatur nachgerührt und das entstandene Amin portionsweise mit Toluol extrahiert. Man trocknete über Kaliumcarbonat und erhielt nach Abtrennung des Toluols 61 g Rohamin von einer Reinheit von 96 % nach gaschromatographischer Analyse.

Beispiel 4 (erfindungsgemäßes Verfahren)

Eine Lösung von 50 g Diamin gemäß Beispiel 2 in 1950 g Chlorbenzol wurde vorgelegt, dann wurden bei 0°C 20 g HCl-Gas unter Rühren über die Flüssigkeitsoberfläche geleitet. Anschließend wurden bei 0°C 50 g Phosgen einkondensiert und nachfolgend innerhalb 60 min bei schwacher Phosgenzugabe (ca. 10 g/h) auf 50°C erwärmt. Innerhalb der nächsten 60 min wurde die Mischung auf 70°C erwärmt und nachfolgend bei einer Phosgenmenge von ca. 20 g/h in 100 min auf Rückfluß gebracht. Unter diesen Bedingungen wurde die Mischung 16 h gerührt. Unreagiertes Phosgen wurde mit Stickstoff ausgeblasen, während die Mischung auf Raumtemperatur abkühlte. Nach Filtration der Mischung wurde das Filtrat vom Lösungsmittel im Vakuum befreit und das Rohprodukt einer Dünnschichtdestillation (Heizbad 210°C, 1 mbar, Übergang bei 180-185°C) unterworfen. Man erhielt 40 g 2,5-Di-(4'-isocyanatobutyl)-3,6-dimethylpyrazin (DIDP) mit einem NCO-Gehalt von 27,9 % (Theorie: 27,8 %). (Ausbeute: 66 %).

Beispiel 5 (erfindungsgemäßes Verfahren)

Einer Lösung von 50 g Diamin gemäß Beispiel 3 in 950 g o-Dichlorbenzol wurde vorgelegt, dann wurden bei 0°C 15 g HCl-Gas unter Rühren über die Flüssigkeitsoberfläche geleitet. Anschließend wurde bei 0°C 50 g Phosgen einkondensiert und nachfolgend innerhalb 50 min bei schwacher Phosgenzugabe (ca. 10g/h) auf 50°C erwärmt. Innerhalb der nächsten 50 min wurde die Mischung auf 70°C erwärmt und nachfolgend bei einer Phosgenmenge von ca. 20 g/h in 60 min auf 135°C gebracht. Bei 135 bis 140°C wurde die Mischung 8 h gerührt. Unreagiertes Phosgen wurde mit Stickstoff ausgeblasen, während die Mischung auf Raumtemperatur abkühlte. Nach Filtration der Mischung wurde das Filtrat vom Lösungsmittel im Vakuum befreit und das Rohprodukt einer Dünnschichtdestillation (Heizbad 210°C, 1 mbar, Übergang bei 180 - 185°C) unterworfen. Man erhielt 33 g 2,5-Di-(4'-isocyanato-butyl)-3,6-dimethylpyrazin (DIDP) mit einem NCO-Gehalt von 27,5 % (Theorie: 27,8 %) als gelbliche, bei Raumtemperatur allmählich kristallisierende Flüssigkeit.

Die oben angegebene Struktur wurde zusätzlich durch Überführung mit Ethanol in das Bisethylurethan bewiesen:

Fp.: 99 - 100°C

| Analyse (%) | C | H | N |
|---|---|---|---|
| gefunden: | 60,8 | 8,7 | 14,1 |
| Theorie: | 60,9 | 8,6 | 14,2 |

(bezogen auf $C_{20}H_{34}N_4O_4$)

Beispiel 6 (Verwendung)

A. Herstellung einer NCO-Prepolymerlösung

Aus 123 g eines Polyesterdiols des Molekulargewichts 1700 auf Basis von Adipinsäure, Hexandiol-1,6 und Neopentylglykol (Gewichtsverhältnis Hexandiol : Neopentylglykol = 65 : 35), 4,2 g eines einwertigen Polyetheralkohols des Molekulargewichts 2150, hergestellt durch Alkoxylierung von n-Butanol unter Verwendung eines Gemischs aus Ethylenoxid und Propylenoxid im Gewichtsverhältnis 80 : 20, und einer Mischung aus 15 g DIDP gemäß Beispiel 4 und 17,9 g 1,6-Diisocyanatohexan wurde bei 100°C durch ca. 90-minütiges Rühren der Ausgangskomponenten ein NCO-endständiges Polyesterprepolymer mit einem NCO-Gehalt von 4,4 % hergestellt. Die Prepolymerschmelze wurde anschließend in Aceton zu einer 46 %igen Lösung gelöst.

B. Herstellung einer wäßrigen, anionischen Polyesterurethanharnstoff-Dispersion

Zu der gesamten acetonischen Lösung des NCO-endständigen

Prepolymers aus A. wurde in zweiminütigem Abstand bei ca. 45°C unter Rühren

1. 5,61 g Isophorondiamin in 20 ml Aceton
2. 0,55 g Hydrazinhydrat
3. 5,39 g 2-Aminoethyl-ß-aminoethansulfonsäure-Natriumsalz (als 20 %ige Lösung in Wasser)

hinzugefügt. Nach weiteren 7 Minuten wurde mit 314 g entsalztem Wasser innerhalb von ca. 3 Minuten dispergiert und das Aceton im Vakuum abgezogen. Die Dispersion wurde mit 160 ml Wasser verdünnt.

Die entstandene anionisch-nichtionische Polyesterurethanharnstoff-Dispersion wies folgende Daten auf:

| | |
|---|---|
| Festkörpergehalt | 30,3 % |
| pH-Wert | 6,8 |
| Teilchengröße | 390 nm |
| $SO_3^-$-Gehalt | 0,7 % |
| Viskosität (25°C) | 6500 mPas (Haake VT-02 Gerät, Spindel # 3). |

Beim Trocknen liefert die Dispersion einen klaren, glänzenden, weichen und hochelastischen Überzug. Das Material ist für Beschichtung von Textil, Leder und anderen Substraten geeignet.

Patentansprüche

1. 2,5-Di-(4'-isocyanatobutyl)-3,6-dimethylpyrazin der Formel

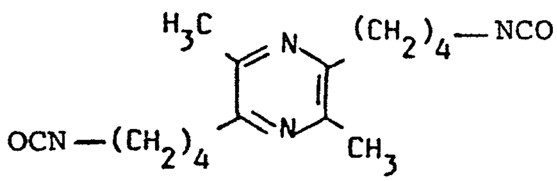

2. Verfahren zur Herstellung von 2,5-Di-(4'-isocyanatobutyl)-3,6-dimethylpyrazin, dadurch gekennzeichnet, daß man 2,5-Di-(4'-aminobutyl)-3,6-dimethylpyrazin oder seine Additionsverbindungen mit Kohlendioxid oder Chlorwasserstoff phosgeniert.

3. Verwendung von 2,5-Di-(4'-isocyanatobutyl)-3,6-dimethylpyrazin als Isocyanatkomponente bei der Herstellung von Polyurethankunststoffen nach dem Isocyanat-Polyadditionsverfahren.

Europäisches Patentamt

**EUROPÄISCHER RECHERCHENBERICHT**

0173870

Nummer der Anmeldung

EP 85 10 9806

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | DE-A-2 800 311 (CIBA-GEIGY AG) * Anspruch 1; Seite 8, Zeilen 1-4 * | 1,3 | C 07 D 241/12<br>C 08 G 18/78 |
| A | EP-A-0 113 044 (BAYER AG) * Ansprüche 4, 5 * | 2,3 | |
| A | DE-A-2 604 831 (BAYER AG) * Ansprüche 2, 3 * | 2,3 | |

RECHERCHIERTE SACHGEBIETE (Int. Cl.4)

C 07 D 241/00
C 08 G 18/00

Der vorliegende Recherchenbericht wurde für alle Patentansprüche erstellt

| Recherchenort | Abschlußdatum der Recherche | Prüfer |
|---|---|---|
| BERLIN | 13-11-1985 | HASS C V F |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument